**Europäisches Patentamt**

**European Patent Office**

**Office européen des brevets**

(11) Veröffentlichungsnummer: **0 014 839**

**A1**

## (12) EUROPÄISCHE PATENTANMELDUNG

(21) Anmeldenummer: **80100187.6**

(22) Anmeldetag: **16.01.80**

(51) Int. Cl.³: **A 61 L 9/20**
**F 21 V 11/02**

(30) Priorität: **18.01.79 DE 2901847**

(43) Veröffentlichungstag der Anmeldung:
**03.09.80 Patentblatt 80/18**

(84) Benannte Vertragsstaaten:
**AT BE CH FR GB IT NL**

(71) Anmelder: **Loher, Alois**
**Talblick 16**
**D-8451 Kümmersbruck 1(DE)**

(72) Erfinder: **Loher, Alois**
**Talblick 16**
**D-8451 Kümmersbruck 1(DE)**

(54) **Vorrichtung zum Keimfreimachen der Luft, unter Verwendung eines vertikal angeordneten stabförmigen UV-Strahlers.**

(57) Vorrichtung zum Keimfreimachen der Luft unter Verwendung eines vertikal angeordneten stabförmigen UV-Strahlers mit einer Anzahl von Lochscheiben, die jeweils durch eine Parallelhalteeinrichtung in einigem Abstand parallel zueinander gehalten sind, wobei die Lochscheiben bezüglich ihres Lochmaßes, ihres Außenmaßes, ihrer Stärke und ihres gegenseitigen Abstandes so aufeinander abgestimmt sind, daß UV-Licht, welches von einem zwischen zwei benachbarten Lochscheiben gelegenen Teilstück dieses UV-Strahlers ausgeht, mit geringem Öffnungswinkel, im wesentlichen nur zwischen diesen beiden benachbarten Lochscheiben, nicht aber zwischen anderen Lochscheiben austritt, wobei die Lochscheiben (21 bis 26) kegelstumpfförmig ausgebildet sind und aus einem UV-Licht undurchlässigen Material bestehen, daß jeweils der Scheibenrand (31) mit dem größeren Durchmesser einer Lochscheibe (z.B. 21) entweder etwa in derselben Ebene oder überlappend liegt zum Scheibenrand (32) mit dem kleineren Durchmesser der benachbarten Lochscheibe (z.B. 21), daß die innere und/oder äußere Oberfläche jeder Lochscheibe (21 bis 26) im Bereich des Scheibenrandes (32) mit dem kleineren Durchmesser einen kleineren Neigungswinkel (w1) zur Stabachse (13) des UV-Strahlers (2) besitzt als im Bereich des Scheibenrandes (31) mit dem größeren Durchmesser, und daß die Lochscheiben (21 bis 26) und der UV-Strahler (2) von einem Rohr (30) aus einem UV-durchlässigen Material Material umgeben sind.

./...

FIG.1

- 1 -

Alois Loher

D — 8451 Kümmersbruck, den 16.01.19
Talblick 16

PA 106

Vorrichtung zum Keimfreimachen der Luft, unter Verwendung eines vertikal angeordneten stabförmigen UV-Strahlers.

Die Erfindung bezieht sich auf eine Blendanordnung für einen stabförmigen UV-Strahler mit einer Anzahl von Blendplatten, die durch eine Parallelhalteeinrichtung in einigem Abstand parallel zueinander gehalten sind, wobei als Blendplatten Lochscheiben vorgesehen sind, die jeweils ein Loch aufweisen, das mindestens zur Durchführung des UV-Strahlers in Richtung seiner Stabachse bemessen ist, wobei die Löcher der einzelnen Lochscheiben axial zueinander ausgerichtet sind, und wobei die Lochscheiben bezüglich ihres Lochmaßes, ihres Außenmaßes, ihrer Stärke und ihres gegenseitigen Abstandes so aufeinander abgestimmt sind, daß UV-Licht, welches - wenn der UV-Strahler axial durch die Löcher gesteckt ist - von einem zwischen zwei benachbarten Lochscheiben gelegenen Teilstück dieses UV-Strahlers ausgeht, mit geringem Öffnungswinkel im wesentlichen nur zwischen diesen beiden benachbarten Lochscheiben, nicht aber zwischen anderen Lochscheiben austritt.

Eine solche Blendanordnung mit stabförmigem UV-Strahler wird in einem Betriebsraum entweder an der Decke angebracht oder auf einem Stativ aufgestellt. Sie dient dazu, Bakterien, die sich in der im Betriebsraum befindlichen Luft und auf der Oberfläche darin befindlicher Gegenstände aufhalten, abzutöten oder an der Vermehrung zu hindern. Wichtig ist es dabei, daß der UV-Strahler praktisch ununterbrochen betrieben werden kann, und zwar auch in Anwesenheit von Menschen und/oder Tieren, ohne diesen zu schaden. Wichtig ist es weiterhin, den UV-Strahler weitgehend staubfrei zu halten, damit die erzeugten UV-Strahlen ungehindert austreten können.

Im Hauptpatent 25 29 166 wird eine Lösung angegeben, die mit ebenen oder leicht gewölbten Lochscheiben ausgerüstet ist. Diese Blendanordnung arbeitet weitgehend zufriedenstellend. Es hat sich jedoch gezeigt, daß in Betriebsräumen mit sehr hohem Staubanfall (z.B. in Viehställen) oder bei langem Betrieb ein gewisser Staubniederschlag auf den einzelnen Lochscheiben zu verzeichnen ist. Dieser Niederschlag reduziert die wirksame Strahlungsleistung. Es hat sich weiter gezeigt, daß dann Reinigungsarbeiten unumgänglich sind, die eine gelegentliche Wartung der Blendanordnung erfordern.

Aufgabe der vorliegenden Erfindung ist es, die eingangs genannte Blendanordnung so auszugestalten, daß sie einen hohen Wirkungsgrad hinsichtlich der abgestrahlten UV-Strahlungsleistung besitzt. Die UV-Strahlung soll dabei möglichst wenig Reflexionsverluste erleiden. Eine Ablagerung von Staub und Feuchtigkeit auf den Lochscheiben soll praktisch vollkommen verhindert werden. Dadurch sollen die Wartungsintervalle vergrößert werden. Allgemein gesprochen soll also die Wirksamkeit der Blendanordnung vergrößert werden.

Die genannte Aufgabe wird erfindungsgemäß dadurch gelöst, daß die Lochscheiben kegelstumpfförmig ausgebildet sind und aus einem UV-Licht undurchlässigen Material bestehen, daß jeweils der Scheibenrand mit dem größeren Durchmesser einer Lochscheibe entweder etwa in derselben Ebene oder überlappend liegt zum Scheibenrand mit dem kleineren Durchmesser der benachbarten Lochscheibe, daß die innere und/oder äußere Oberfläche jeder Lochscheibe im Bereich des Scheibenrandes mit dem kleineren Durchmesser einen kleineren Neigungswinkel zur Stabachse des UV-Strahlers besitzt als im Bereich des Scheibenrandes mit dem größeren Durchmesser, und daß die Lochscheiben und der UV-Strahler von einem Rohr aus einem UV-durchlässigen Material umgeben sind.

Das besagte UV-durchlässige Rohr, das insbesondere aus Quarzglas bestehen kann, sorgt bei hermetischem Abschluß dafür, daß die Lochscheiben und der UV-Strahler vor Staub und Feuchtigkeit geschützt werden. Selbstverständlich ist an beiden Enden dieses Rohres dazu ein dichtender Abschluß erforderlich. Die kegelstumpfförmige Ausbildung der einzelnen Lochscheiben dient zwei Zwecken: Zum einen wird die UV-Strahlung von der inneren Oberfläche der Lochscheiben am direkten Austritt in die Umgebung des UV-Strahlers gehindert. Die Lochscheibe wirkt insoweit als Blende. Da sie aber gut reflektierend ausgebildet ist, sorgt sie gleichzeitig für eine Weiterleitung der auffallenden UV-Strahlung. Zum anderen dient die äußere Oberfläche einer jeden Lochscheibe als reflektierendes Medium für die Reflexion nach außen. Dadurch ist eine gute Strahlungsausnutzung gewährleistet. Die unterschiedliche Neigung im Bereich des oberen und des unteren Scheibenrandes verhindert unerwünschte Streustrahlung. Der hermetische Abschluß des Rohres gewährleistet gleichzeitig, daß die reflektierenden Oberflächen nicht blind werden können.

- 4 -    PA 106

Eine bevorzugte Weiterbildung der Blendanordnung ist dadurch gekennzeichnet, daß die unterschiedlich geneigte Oberfläche mindestens einer Lochscheibe - im Längsschnitt zur Stabachse gesehen - mindestens zwei unterschiedlich geneigte Stufen besitzt. Dadurch ist gewährleistet, daß das zwischen zwei benachbarten Lochscheiben austretende UV-Licht eine verhältnismäßig große Durchtrittsöffnung vorfindet. Die einzelnen Bereiche zwischen den Stufen können hierbei einen geradlinigen Verlauf besitzen. Eine solche Ausführung ist relativ einfach herstellbar.

Um aber die Streustrahlung noch weiter zu reduzieren und den Wirkungsgrad noch weiter zu erhöhen, kann nach einer weiteren Ausführungsform vorgesehen sein, daß die einzelnen Bereiche - bei Blick senkrecht auf die Stabachse - einen konkav gekrümmten Verlauf besitzen.

Eine leichte Herstellbarkeit der Blendanordnung ist insbesondere gewährleistet, wenn die Lochscheiben bezüglich der Stabachse des UV-Strahlers rotationssymmetrisch ausgebildet sind.

Weitere vorteilhafte Ausführungsformen sind in den Unteransprüchen gekennzeichnet.

Bei der erfindungsgemäßen Blendanordnung kann nur eine geringe Streustrahlung auftreten. Bei einem derartigen Gerät an der Decke kann die Strahlung horizontal oder nach oben gerichtet austreten, und bei einem Stativgerät kann die UV-Strahlung horizontal oder nach unten gerichtet sein. Damit wird eine Gefährdung von Lebewesen ausgeschaltet.

Ausführungsbeispiele der Erfindung werden im folgenden anhand von fünf Figuren näher erläutert. Es zeigen:

Figur 1 eine an der Decke aufgehängte Blendanordnung für einen stabförmigen UV-Strahler in einer seitlichen Ansicht,

Figur 2 einen Querschnitt durch die Blendanordnung nach Figur 1,

Figur 3 einen Längsschnitt der Blendanordnung in vergrößerter Darstellung, wobei verschiedene Ausführungsformen von kegelstumpfförmig ausgebildeten Lochscheiben dargestellt sind,

Figur 4 einen Blick von oben auf eine Lochscheibe, die mit drei Stützarmen zur Halterung der benachbarten oberen Lochscheibe verstehen ist, und

Figur 5 eine perspektivische Ansicht der Lochscheibe nach Figur 4.

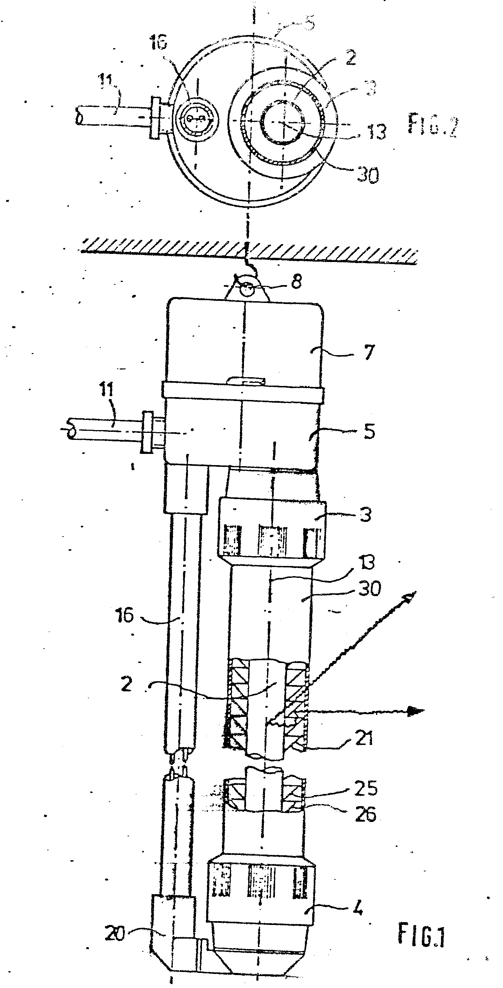

Figur 1 zeigt in einer Seitenansicht einen stabförmigen UV-Strahler 2 runden Querschnitts, der von einer ersten und einer zweiten Fassung 3 bzw. 4 in vertikaler Lage lösbar gehalten ist. Die erste Fassung 3 ist an einem Gehäuse 5 verschraubt. Das Gehäuse 5, dessen Deckel mit 7 bezeichnet ist, enthält die elektrischen Zubehörteile für den UV-Strahler 2, u.a. die Starterdrosselspule. Zum Aufhängen des Gehäuses 5 an der Decke eines Betriebsraumes ist eine Öse 8 am Deckel 7 vorgesehen. In das Gehäuse 5 führt von der Seite ein Zuleitungskabel 11, das den UV-Strahler 2 mit elektrischer Energie versorgt. Die Stabachse des UV-Strahlers 2 ist mit 13 bezeichnet.

Vom Gehäuse 5 aus führt parallel zum UV-Strahler 2 nach unten ein Halterohr 16 aus Metall, das mit der zweiten Fassung 4 durch ein Winkelstück 20 verbunden ist. Durch dieses Halterohr 16 und das Winkelstück 20 läuft ein elektrisches Anschlußkabel zur zweiten Fassung 4.

Um die Strahlung des UV-Strahlers 2 weitgehend nach unten hin abzublenden, ist eine Blendanordnung vorgesehen.

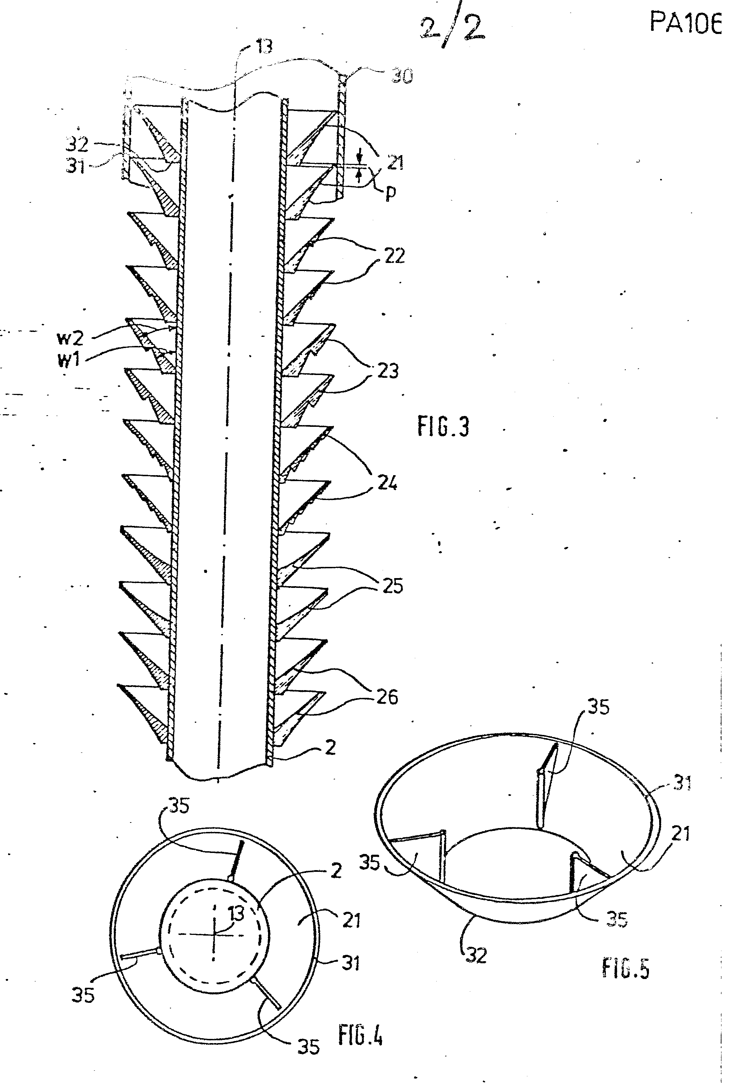

Diese umfaßt, wie aus der teilweise aufgebrochenen Darstellung in Figur 1 ersichtlich ist, eine größere Anzahl von Lochscheiben, von denen lediglich drei Lochscheiben 21, 25 und 26 mit Bezugszeichen versehen sind. Alle Lochscheiben sind kegelstumpfförmig ausgebildet und konzentrisch zur Stabachse 13 angeordnet, wobei bei jeder Lochscheibe der Scheibenrand mit dem kleineren Durchmesser nach unten weist. Sie werden durch Stege, wie später anhand der Figuren 4 und 5 verdeutlicht wird, parallel zueinander und konzentrisch zum UV-Strahler 2 gehalten. Die Lochscheiben 21 bis 26 und der UV-Strahler 2 sind von einem Rohr 30 aus einem UV-durchlässigen Material umgeben. Es handelt sich dabei um ein Schutzrohr, das insbesondere aus Quarzglas bestehen kann. Von dem UV-Strahler 2 ausgehendes UV-Licht kann entweder direkt oder nach Reflexion auf einer inneren oder äußeren Oberfläche der Lochscheiben 21 bis 26 durch das Rohr 30 in den Außenraum austreten. In Figur 1 sind zwei vom UV-Strahler 2 ausgehende Strahlen eingezeichnet, die dies veranschaulichen. Das Rohr 30 reicht zu den Fassungen 3 und 4, so daß die Lochscheiben gegen Staubeinfall hermetisch abgeschlossen sind.

Aus Figur 2 ist ersichtlich, daß das Rohr 30 und damit auch die einzelnen Lochscheiben 21 bis 26 konzentrisch zur Stabachse 13 und zum UV-Strahler angeordnet sind.

Aus Figur 3 sind sechs unterschiedliche Ausführungsformen der Lochscheiben 21 bis 26 zu entnehmen. Allen Lochscheiben 21 bis 26 ist gemeinsam, daß sich ihre Dicke nach außen hin - zumindest abschnittsweise - verringert. Dabei ist der zur Stabachse 13 gemessene Neigungswinkel der Außenfläche jeweils außen größer als innen in der Nähe der Stabachse 13. Es sind jeweils zwei identische

Lochscheiben 21 bis 26 übereinander dargestellt. In der
Praxis wird man jedoch alle Lochscheiben einer Blendanordnung in gleicher Weise ausbilden. Alle Lochscheiben
21 bis 26 sind zur Stabachse 13 rotationssymmetrisch.
Außerdem sind alle Lochscheiben 21 rundum mit einer
gut UV-Strahlung reflektierenden Oberfläche versehen.

An den beiden oben eingezeichneten Lochscheiben 21 wird
deutlich, daß der Scheibenrand 31 mit dem größeren Durchmesser der unteren Lochscheibe 21 überlappend liegt zum
Scheibenrand 32 mit dem kleineren Durchmesser der benachbarten oberen Lochscheibe 21. Der Überlappungsabstand
ist mit p bezeichnet. Dieser Abstand p ist so gewählt,
daß kein UV-Licht direkt nach unten oder horizontal austreten kann.

Aus Figur 3 ist weiter zu entnehmen, daß bei den beiden
Lochscheiben 21 jeweils die innere Kegelmantellinie
(entsprechend der Innenfläche) gerade ausgeführt und
die äußere Kegelmantellinie (entsprechend der Außenfläche) durchgehend konkav gekrümmt ist. Der Neigungswinkel der inneren Kegelmantellinie beträgt etwa 45°.
Demgegenüber ist bei den beiden Lochscheiben 22 jeweils
die innere Kegelmantellinie zwar ebenfalls gerade, die
äußere Kegelmantellinie jedoch in einer Stufe abgesetzt.
Beide Teilbereiche der äußeren Oberfläche sind konkav
gekrümmt. Dadurch wird der für das UV-Licht wirksame
Lichtspalt vergrößert. Die äußere Oberfläche der Lochscheiben 22 kann als eine stufenweise Annäherung an die
äußere Oberfläche der Lochscheiben 21 angesehen werden.

Auch bei den beiden Lochscheiben 23 ist jeweils die
innere Kegelmantellinie gerade ausgeführt. Die äußere
Kegelmantellinie ist jedoch - wie bei den Lochscheiben
22 - mit einer (einzigen) Stufe versehen. Allerdings
sind die beiden Teilbereiche der äußeren Oberfläche hier nicht gekrümmt, sondern gerade ausgeführt. Es ist ersichtlich, daß der innere Teilbereich steiler verläuft als der äußere Teilbereich. Mit anderen Worten: Der Neigungswinkel w1 zur Stabachse 13 ist am inneren Teilbereich kleiner als der Neigungswinkel w2 am äußeren Teilbereich. Die äußere Oberfläche der Lochscheiben 23 kann somit als eine Annäherung an die äußere Oberfläche der Lochscheiben 22 angesehen werden.

Bei den Lochscheiben 24 sind die inneren Kegelmantellinien ebenfalls gerade ausgeführt. Auch die äußere Kegelmantellinie ist gerade geführt, jedoch mehrfach gestuft. Insgesamt ergeben sich bei jeder Lochscheibe 24 fünf äußere Teiloberflächen oder Teilbereiche. Im dargestellten Schnitt ist also die äußere Kegelmantellinie sägezahnförmig. Die Steilheit der einzelnen Teilbereiche ist in weitgehender Annäherung zur Krümmung entsprechend der äußeren Kegelmantellinie der Lochscheiben 21 gewählt. Diese Ausführungsform ist bevorzugt.

Bei den Lochscheiben 25 ist wiederum eine nach außen abnehmende Dicke zu verzeichnen. Jedoch ist hier jeweils die innere Kegelmantellinie durchgehend konkav gekrümmt und die äußere Kegelmantellinie gerade ausgeführt. Stufen sind hier nicht vorgesehen, können aber vorhanden sein.

Bei den Lochscheiben 26 ist vorgesehen, daß sowohl die innere als auch die äußere Kegelmantellinie konkav gekrümmt ist. Die Krümmung der inneren und der äußeren Oberfläche kann dabei unterschiedlich sein. Auch hier können Stufen vorhanden sein.

In einer erprobten Ausführungsform betrug der Innendurchmesser des Rohres 30 etwa 47 mm und seine Wandstärke 1,5 mm. Der UV-Strahler 2 besaß einen Außendurchmesser von 28 mm. Die Lochscheiben 21 bis 26 waren entsprechend den Lochscheiben 24 mit jeweils insgesamt 5 Stufen ausgeführt. Die einzelnen Stufen besaßen dabei einen Abstand von etwa 2 mm. Die Höhe einer Lochscheibe - in Richtung der Stabachse 13 gemessen - betrug etwa 9 mm. Die Erfindung ist auf diese Maßangaben natürlich nicht beschränkt.

Aus den Figuren 4 und 5 geht hervor, in welcher Weise die einzelnen Lochscheiben 21 bis 26, die natürlich alle gleich ausgeführt sein können, parallel zueinander gehalten werden können. Die dargestellte Lochscheibe 21 weist innen im Bereich des Scheibenrandes 31 mit dem größeren Durchmesser insgesamt drei um 120° gegeneinander versetzte Stützarme 35 auf, die in Richtung auf den UV-Strahler 2 weisen. Diese Stützarme 35 sind zu innen angearbeiteten, dreieckförmigen Stützflächen ergänzt. Jeder Stützarm 35 ist gegenüber dem oberen Scheibenrand 31 etwas nach unten abgesetzt, wodurch der Überlappungsabstand p zustandekommt. Auf den drei Stützarmen 35 liegt der Scheibenrand 32 mit dem kleineren Durchmesser der benachbarten oberen Lochscheibe 21 auf. Durch den UV-Strahler 2 und das Rohr 30 werden die einzelnen Lochscheiben 21 konzentrisch und durch die Stützarme 35 parallel zueinander gehalten. Die Stützarme 35 haben somit die Funktion von Stütz- und Führungsstegen. Prinzipiell können entsprechende Stützarme oder Stützflächen auch außen an der Lochscheibe 21 angeordnet sein.

Die Lochscheiben 21 bis 26 können aus einem Kunststoff bestehen, der allseitig mit einem die UV-Strahlung gut reflektierenden Überzug wie Silber oder bevorzugt Aluminium versehen ist. Bei einer solchen Ausgestaltung der Lochscheiben 21 bis 26 können die Stützarme 35, besser noch die erwähnten Stützflächen, zusammen mit den kegelstumpfförmigen Lochscheiben in einem Spritzvorgang hergestellt werden. Das führt zu einer besonders einfachen Herstellung. Prinzipiell können die Lochscheiben auch aus massivem Aluminium bestehen.

16 Patentansprüche
5 Figuren

Algier ....         D - 8451 Kümmersbruch, den 25.01.1980
                    Talblick 16

PA 105

Patentansprüche

1. Vorrichtung zum Keimfreimachen der Luft unter Verwendung eines vertikal angeordneten stabförmigen UV-Strahlers mit einer Anzahl von Lochscheiben, die jeweils durch eine Parallelhalteeinrichtung in einigem Abstand parallel zueinander gehalten sind, wobei die Lochscheiben bezüglich ihres Lochmaßes, ihres Außenmaßes, ihrer Stärke und ihres gegenseitigen Abstandes so aufeinander abgestimmt sind, daß UV-Licht, welches von einem zwischen zwei benachbarten Lochscheiben gelegenen Teilstück dieses UV-Strahlers ausgeht, mit geringem Öffnungswinkel im wesentlichen nur zwischen diesen beiden benachbarten Lochscheiben, nicht aber zwischen anderen Lochscheiben austritt,

..., dadurch gekennzeichnet, daß die Lochscheiben (21 bis 26) kegelstumpfförmig ausgebildet sind und aus einem UV-Licht undurchlässigen Material bestehen, daß jeweils der Scheibenrand (31) mit dem größeren Durchmesser einer Lochscheibe (z.B. 21) entweder etwa in derselben Ebene oder überlappend liegt zum Scheibenrand (32) mit dem kleineren Durchmesser der benachbarten Lochscheibe (z.B. 21), daß die innere und/oder äußere Oberfläche jeder Lochscheibe (21 bis 26) im Bereich des Scheibenrandes (32) mit dem kleinern Durchmesser einen kleineren Neigungswinkel (w1) zur Stabachse (13) des UV-Strahlers (2) besitzt als im Bereich des Scheibenrandes (31) mit dem größeren Durchmesser, und daß die Lochscheiben (21 bis 26) und der UV-Strahler (2) von einem
Rohr (30) aus einem UV-durchlässigen Material umgeben
sind.

2. Vorrichtung   nach Anspruch 1, d a d u r c h   g e-
k e n n z e i c h n e t, daß die nicht unterschiedlich
geneigte Oberfläche mindestens einer Lochscheibe (21 bis
24) - im Längsschnitt zur Stabachse (13) gesehen -
einen geradlinigen Verlauf besitzt.

3. Vorrichtung   nach Anspruch 2, d a d u r c h   g e-
k e n n z e i c h n e t, daß der Neigungswinkel dieser
Oberfläche zur Stabachse (13) etwa 45° beträgt.

4. Vorrichtung   nach einem der Ansprüche 1 bis 3, d a-
d u r c h   g e k e n n z e i c h n e t, daß die unterschiedlich geneigte  Oberfläche mindestens einer Lochscheibe (21, 22, 25, 26) - im Längsschnitt zur Stabachse
(13) gesehen - einen durchgehend gekrümmten Verlauf besitzt.

5. Vorrichtung   nach einem der Ansprüche 1 bis 3, d a-
d u r c h   g e k e n n z e i c h n e t, daß die unterschiedlich geneigte Oberfläche mindestens einer Lochscheibe (22, 23, 24) - im Längsschnitt zur Stabachse
(13) gesehen - mindestens zwei unterschiedlich geneigte
Teilbereiche besitzt, die durch eine Stufe voneinander
getrennt sind.

6. Vorrichtung   nach Anspruch 5, d a d u r c h   g e-
k e n n z e i c h n e t, daß die Teilbereiche in mindestens einer Lochscheibe (23, 24) jeweils einen geradlinigen Verlauf besitzen.

7 Vorrichtung nach Anspruch 5, d a d u r c h g e - k e n n z e i c h n e t , daß die Teilbereiche der Lochscheibe (22) einen - bei Blick senkrecht auf die Stab- achse (13) - konkav gekrümmten Verlauf besitzen.

8. Vorrichtung nach einem der Ansprüche 5 bis 7, d a - d u r c h g e k e n n z e i c h n e t , daß die Länge jedes Teilbereichs bei der Lochscheibe (24) etwa 2 mm beträgt.

9. Vorrichtung nach einem der Ansprüche 5 bis 8, d a - d u r c h g e k e n n z e i c h n e t , daß die Anzahl der Teilbereiche pro Lochscheibe (24) mit abgestufter Oberfläche gleich 5 ist.

10. Vorrichtung nach einem der Ansprüche 1 bis 9, d a d u r c h g e k e n n z e i c h n e t , daß die Lochscheiben (21 bis 26) bezüglich der Stabachse (13) des UV-Strahlers (2) rotationssymmetrisch ausgebildet ist.

11. Vorrichtung nach einem der Ansprüche 1 bis 10, d a d u r c h g e k e n n z e i c h n e t , daß beide Oberflächen einer jeden Lochscheibe (21 bis 26) für UV-Strahlung gut reflektierend ausgebildet sind.

12. Vorrichtung nach Anspruch 11, d a d u r c h g e k e n n z e i c h n e t , daß die Lochscheiben (21 bis 26) massiv ausgebildet sind und aus Aluminium bestehen.

13. Vorrichtung nach Anspruch 11, d a d u r c h g e k e n n z e i c h n e t , daß die Lochscheiben (21 bis 26) aus einem Kunststoff bestehen, der mit einem die UV-Strahlung gut reflektierenden Überzug versehen ist.

14. Vorrichtung nach einem der Ansprüche 1 bis 13, d a d u r c h   g e k e n n z e i c h n e t, daß jede Lochscheibe (21 bis 26) im Bereich des Scheibenrandes (31) mit dem größeren Durchmesser mindestens einen in Richtung auf den UV-Strahler (2) weisenden Stützarm (35) zur Halterung des Scheibenrandes (32) mit dem kleineren Durchmesser der benachbarten Lochscheibe (21 bis 26) besitzt (Figur 4 und 5).

15. Vorrichtung nach einem der Ansprüche 1 bis 13, d a d u r c h   g e k e n n z e i c h n e t, daß jede Lochscheibe (21 bis 26) im Bereich des Scheibenrandes (31) mit dem kleineren Durchmesser mindestens einen in Richtung auf den UV-Strahler (2) weisenden Stützarm (35) zur Halterung des Scheibenrandes (32) mit dem größeren Durchmesser der benachbarten Lochscheibe (21 bis 26) besitzt (Figur 4 und 5).

16. Vorrichtung nach Anspruch 15 oder 16, d a d u r c h   g e k e n n z e i c h n e t, daß als Stützarme (35) mindestens drei Stege vorgesehen sind (Figur 4 und 5).

1/2

0014839

PA106

FIG.2

FIG.1

FIG.3

FIG.4

FIG.5

# EUROPÄISCHER RECHERCHENBERICHT

**Europäisches Patentamt**

Nummer der Anmeldung

EP 80 10 0187

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| Y | GB - A - 308 625 (A.F. BERRY) <br> * Seite 4, Anspruch 1; Abbildung 4 * | 1-16 |
| X | GB - A - 202 009 (A.N. HAWORTH) <br> * Seite 3, Anspruch 1; Abbildung 7 * | 1-16 |
| | FR - A - 2 194 447 (PHILIPS) <br> * Seite 4, Anspruch 1; Abbildung 1 * | 1 |
| D | FR - A - 2 316 534 (H. SOMMER) <br> * Abbildungen 1,12 * <br> & DE - A - 2 529 166 | 1 |
| | US - A - 2 617 918 (W.A. FOSTER) | 1-16 |
| | US - A - 2 363 374 (E.R. WEYER) | 1-16 |

**KLASSIFIKATION DER ANMELDUNG (int. Cl.)**

A 61 L 9/20
F 21 V 11/02

**RECHERCHIERTE SACHGEBIETE (int. Cl.)**

A 61 L 9/20
        2/10
F 21 V 11/02

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

X Der vorliegende Recherchenbericht wurde für alle Patentansprüche erstellt

| Recherchenort. | Abschlußdatum der Recherche | Prüfer |
|---|---|---|
| Den Haag | 20-05-1980 | PELTRE |

EPA form 1503.1   06.78